(19) Europäisches Patentamt
European Patent Office
Office européen des brevets

(11) EP 2 380 944 A1

(12) EUROPEAN PATENT APPLICATION

(43) Date of publication:
26.10.2011 Bulletin 2011/43

(51) Int Cl.:
C09K 11/77 (2006.01)    A61N 5/00 (2006.01)
A61N 5/06 (2006.01)    H01L 33/00 (2010.01)

(21) Application number: 10160399.1

(22) Date of filing: 20.04.2010

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA ME RS

(71) Applicants:
• Koninklijke Philips Electronics N.V.
5621 BA Eindhoven (NL)
• Philips Intellectual Property & Standards GmbH
20099 Hamburg (DE)

(72) Inventors:
• Juestel, Thomas,
5600 AE, Eindhoven (NL)
• Scholl, Robert, P.,
5600 AE, Eindhoven (NL)

(74) Representative: Damen, Daniel Martijn
P.O. Box 220
5600 AE  Eindhoven (NL)

(54) **Light source for emitting infrared radiation, particularly for medical skin irradiation**

(57)    A light source (1) for emitting infrared radiation which may be specifically used for irradiating living tissue in medical applications is proposed. The light source (1) comprises a light emitter (3) comprising for example LEDs (9) for emitting non-infrared radiation e.g. in a spectral range of between 370 and 900 nm. The light source (1) further comprises a wavelength converting element (5) for converting non-infrared radiation into infrared radiation preferably in a wavelength spectrum excluding wavelength ranges in which water significantly absorbs infrared radiation. The proposed infrared light source (1) is highly efficient due to its radiation conversion principle and may provide infrared radiation with specific characteristics allowing deep penetration into living tissue.

Fig. 1

EP 2 380 944 A1

**Description**

FIELD OF THE INVENTION

The present invention relates to a light source for emitting infrared radiation and to using such light source for irradiating living tissue such as medical irradiation of skin.

BACKGROUND OF THE INVENTION

It has been observed that irradiating living tissue with infrared radiation may be of medical benefit. Particularly via irradiation with light of the near infrared (NIR) spectral range thermal energy may be introduced deep into the living tissue. For example, the skin of a person may be illuminated with NIR light thereby efficiently heating the skin as well as the tissue underneath the skin.

However, it has been observed that with conventional NIR lamps the tissue may be heated inhomogeneously. Particularly, local overheating in regions close to the surface of the skin may occur.

SUMMARY OF THE INVENTION

Accordingly, there may be a need for an improved light source for emitting infrared radiation providing improved irradiation characteristics. Furthermore, such light source should be easy to manufacture, preferably at low cost, and easy and secure to use, particularly for medical applications.

According to an aspect of the present invention, a light source for emitting infrared radiation is proposed to comprise a light emitter for emitting non-infrared radiation, such as e.g. electromagnetic radiation with a wavelength below approximately 800 nm, and a wavelength converting element which converts non-infrared radiation emitted by the light emitter into infrared radiation, preferably in the near infrared spectral range of e.g. approximately 800-1700 nm.

Therein, the wavelength converting element may be adapted to convert the non-infrared radiation into infrared radiation within a wavelength spectrum excluding wavelength ranges in which water ($H_2O$) significantly absorbs radiation. Particularly, such wavelength spectrum may consist of a first wavelength range below 950 nm, a second wavelength range from 1000-1100 nm, a third range from 1200-1350 nm and a fourth range from 1500-1700 nm.

The wavelength converting element may comprise a luminescent material. The luminescent material may comprise activator ions integrated into a matrix material. The activator ions may comprise $F_e^{3+}$, $pr^{3+}$, $Nd^{3+}$, $Sm^{3+}$, $Er^{3+}$, $Tm^{3+}$ or $Yb^{3+}$ ions. The luminescent material may be provided in the form of a monocrystalline structure, a polycrystalline structure, a polycrystalline ceramic structure, a micro-scale powder or a nano-scale powder. The matrix material may comprise a crystalline material, a ceramic material, a glass-ceramic material, a glass-like material or a polymer material.

The wavelength converting element may be provided as a screen which is transparent or translucent and which is arranged in a light path from the light emitter towards a light application location.

The light emitter of the light source may be one or more light emitting diodes (LED). Advantageously, a plurality of LEDs form an LED panel. Using for example such LED panel, the light emitter and the radiation converting element of the light source may be adapted such as to emit infrared radiation with a power density between 1 and 10000 mW/cm2.

The proposed light source may be used for irradiating living tissue with infrared radiation, particularly for medical irradiation of skin.

An object of the present invention or some of its embodiments may rely on the observation that it may be difficult to provide a light emitter emitting itself infrared radiation with an advantageous wavelength spectrum or acceptable efficiency and that it may be, instead, easier or more efficient to generate a desired infrared wavelength spectrum by emitting light from a light emitter with a non-infrared wavelength spectrum and then converting this non-infrared wavelength spectrum into infrared wavelength radiation using a specific wavelength converting element.

A further observation is that living tissue frequently comprises a high content of water significantly absorbing infrared radiation within specific spectral ranges. The infrared absorption due to the water content may prevent radiation from reaching deeper regions within the tissue. Furthermore, the portion of the irradiating infrared radiation heavily absorbed by the water-comprising skin tissue close to the skin surface may excessively heat such superficial skin regions thereby possibly reducing a patient's comfort or even damaging the patient's skin. Thus, it may be advantageous to provide an infrared light source emitting light with a wavelength spectrum in which the wavelength ranges in which water significantly absorbs infrared radiation are excluded.

Details of embodiments of the light source and particularly of the wavelength converting element thereof adapted to provide such radiation characteristics are given further below.

BRIEF DESCRIPTION OF THE DRAWINGS

Features and advantages of the present invention will be further described with reference to specific embodiments as shown in the accompanying figures but to which the invention shall not be limited.

Fig. 1 shows a light source according to an embodiment of the present invention.
Fig. 2 shows a wavelength spectrum of sunlight together with water absorption bands in the near infrared spectral range.
Figs. 3 to 6 show emission spectra of luminescent materials usable in a wavelength converting elements of a light source according to embodiments of the present invention.

Features shown in the drawings are schematic only and are not to scale.

DETAILED DESCRIPTION OF EMBODIMENTS

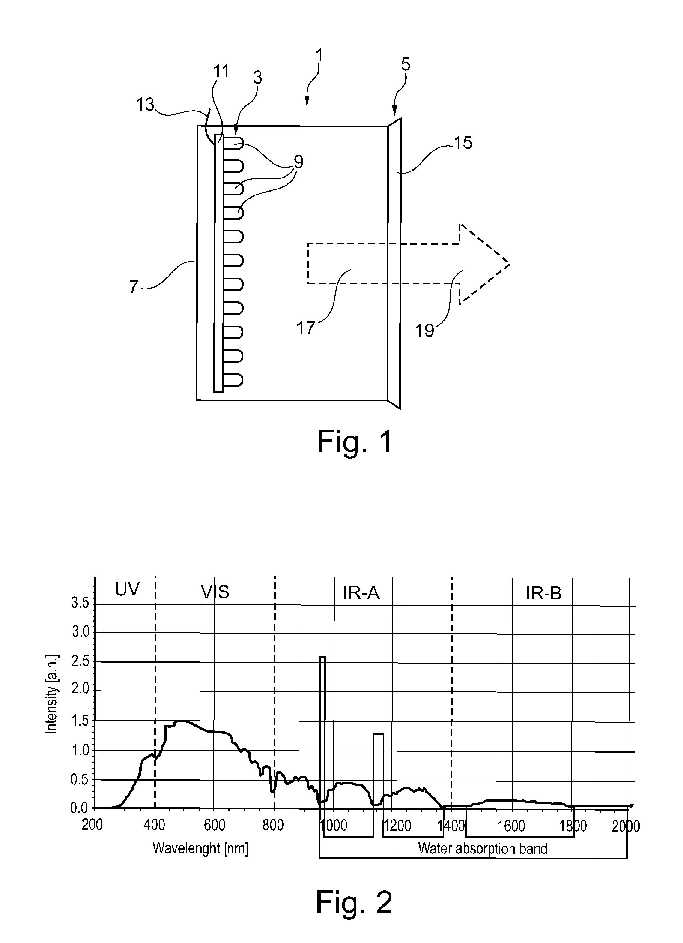

Fig. 1 shows a light source 1 according to an embodiment of the present invention. The light source 1 comprises a light emitter 3 for emitting non-infrared radiation and a wavelength converting element 5 for converting the non-infrared radiation emitted by the light emitter 3 into infrared radiation. The light emitter 3 is accommodated within an opaque or reflecting housing 7. The light emitter 3 comprises a plurality of light emitting diodes LED 9 arranged as an LED panel on a substrate 11 and being supplied with electrical energy via a supply line 13. Light emitted by the LEDs 9 can exit the housing 7 through the transparent or translucent screen 15 serving as a wavelength converting element 5. As explained in further detail below, the screen 15 is adapted to convert the non-infrared light 17 emitted by the LEDs 9 into infrared light 19, preferably in the near infrared spectral range, as indicated by the dotted arrow in Fig. 1. This NIR light may then be used to irradiate living tissue such as the skin of a person.
Fig. 2 schematically shows a typical wavelength spectrum of sunlight.
Compared to black body radiation, specific portions of the spectrum are missing, particularly in the infrared-A range (760-1400 nm) and the infrared-B range (1400-3000 nm) comprised in the near infrared (NIR) spectral range. These missing spectral portions mainly result from absorption of the sunlight by droplets of water or water vapour comprised in the atmosphere. Water typically absorbs in water absorption bands at about 960 nm, about 1150 nm, about 1400 nm and between about 1800 and 2000 nm, as schematically shown in Fig. 2.
Human skin or human tissue also comprises a significant amount of water. Accordingly, electromagnetic radiation in the spectral range of the water absorption bands is significantly absorbed within the outer skin layers (epidermis or dermis). In order to be able to reach deeper skin layers (e.g. in the subcutis) or even tissue underneath the skin, electromagnetic radiation should be used which is not or hardly absorbed by water.
Conventional NIR lamps are based on emission of electromagnetic radiation over a large spectral range, including NIR radiation, and then possibly filtering out the NIR radiation by absorbing radiation of other wavelength. For example, a halogen lamp can be used as a radiation source and the light emitted by the halogen lamp may subsequently be filtered using for example an orange-filter, removing at least the blue from the halogen spectrum, before irradiating the skin of the patient. However, due to the filtering and the radiation absorption occurring therewith, a significant portion of the radiation emitted by the halogen lamp is wasted thereby reducing the efficiency of the NIR lamp.
A NIR light source according to embodiments of the present invention relies on a completely different principle. Instead of filtering non-desired radiation emitted by a wide spectrum light emitter this radiation is converted by a wavelength converting element into radiation within the desired infrared wavelength spectrum. Using such conversion principle instead of filtering via absorption allows achieving significantly higher overall efficiencies for the NIR light source.
An advantage of the invention is that infrared radiation may be generated from non-infrared emitting radiation sources. As a light emitter 3 for emitting non-infrared radiation various types of light emitters may be used. Such light emitters 3 may be adapted to emit radiation exclusively or at least preferably within a non-infrared spectral range, i.e. a spectral range with wavelengths below 800 nm such as for example within the visible spectral range VIS. It may be preferred that the light emitter emits radiation within a relatively narrow wavelength band having a width of for example less than 400 nm, preferably less than 100 nm in order to enhance the conversion efficiency of the wavelength converting element.
For example, a light emitting diode 9 or a plurality of light emitting diodes 9 arranged on a substrate 11 may be advantageously used as a light emitter 3. LEDs typically have a high conversion efficiency from electrical energy to light, thereby contributing to the overall efficiency of the light source 1. Furthermore, LEDs may be adapted to emit radiation in a small spectral wavelength band. Specifically, LEDs may be adapted to emit in desired wavelength bands in order to improve conversion of the original non-infrared radiation into infrared radiation within small spectral wavelength bands in order to thereby improve a penetration depth of the generated infrared radiation. For example, LEDs based on (Al, Ga, In)(P, As) emitting at approximately between 600 and 900 nm or LEDs based on (Al, Ga, In)N emitting approximately between 370 and 500 nm may be used for the light emitter 3.

It may be noted that, while LEDs emitting in a spectral range of 600-950 nm may be commercially available, no LEDs emitting in a spectral range wavelengths above 1000 nm are currently commercially available. Thus, the wavelength converting principle underlying the light source according to embodiments of the present invention currently seems to be the only way to use LEDs for efficiently generating infrared radiation.

The non-infrared radiation emitted by the light emitter 3 may then be converted into infrared radiation using the screen 15 acting as a wavelength converting element 5. The screen 15 and particularly its material may be specifically adapted such that the incoming non-infrared radiation is efficiently converted into infrared radiation preferably in a wavelength spectrum excluding wavelength ranges corresponding to the water absorption bands, i.e. excluding wavelength ranges in which water droplets or water vapour e.g. in the earth atmosphere have maximum infrared radiation absorption. In this context, "having maximum infrared radiation absorption" may mean that in these maximum absorption wavelength ranges the absorption of water, as comprised e.g. in the form of droplets in the atmosphere, is for example 50% more or preferably 100% more than in neighbouring wavelength ranges outside these maximum absorption wavelength ranges. In other words, water has a maximum absorption in spectral ranges around about 960 nm, about 1150 nm, about 1400 nm and between about 1800 and 2000 nm plus/minus e.g.1 to 5 % relative or plus/minus e.g. 10nm to 100nm absolute. For example, the material of the screen 15 may be selected such as to convert the incoming non-infrared radiation from the light emitter 3 into outgoing infrared radiation only within or at least predominantly within wavelength ranges outside the water absorption bands such as e.g. within a wavelength range of between 600 and 950 nm, preferably between 700 and 900 nm and/or within a wavelength range of between 1000 and 1100 nm, preferably between 1030 and 1080 nm, and/or within a wavelength range of between 1200 nm and 1350 nm, preferably between 1220 and 1280 nm, and/or within a wavelength range between 1500 and 1700 nm, preferably between 1550 and 1650 nm.

In order to achieve such advantageous wavelength converting characteristics, the screen 15 of the wavelength converting element 5 may be provided with a luminescent material. Such luminescent materials may include phosphorescent materials as well as fluorescent materials. Frequently, luminescent materials are also referred to as "phosphors" although not specifically referring to phosphor as a chemical element but to all phosphorescent or fluorescent materials. The luminescent material may comprise activator ions which emit in the near infrared spectral range. Such activator ions may be iron ions ($Fe^{3+}$), praseodymium ions ($Pr^{3+}$), neodymium ions ($Nd^{3+}$), samarium ions ($S^{m3+}$), erbium ions ($Er^{3+}$), thulium ions ($Tm^{3+}$) and/or ytterbium ions ($Yb^{3+}$). To obtain high conversion efficiency, these wavelength converting activator ions may be incorporated into a crystalline environment with low phonon frequencies, e.g. sesquioxides, aluminates, garnets, sulfides, selenides, tellurides, titanates, zirconates, hafnates, tungstates, molybdates, or fluorides.

Particularly, luminescent compositions with the following formulas may be used in a wavelength converting element:

$$AE_{1-2z}(Ti_{1-a-b}Zr_aHf_b)O_3:Ln_zNa_z$$

$$(La_{1-x-y-z}Gd_xY_y)_2(Ti_{1-a-b}Zr_aHf_b)_2O_7:Ln_z$$

$$(La_{1-x-y-z}Gd_xY_y)_2O_3:Ln_z$$

$$(Lu_{1-x-y-z}Gd_xY_y)_3Al_5O_{12}:Ln_z$$

$$(La_{1-x-y-z}Gd_xY_y)MgAl_{11}O_{19}:Ln_z$$

$$(La_{1-x-y-z}Gd_xY_y)AlO_3:Ln_z$$

$$Sr_{1-2z}Al_{12}O_{19}:Ln_zNa_z$$

$$Ca(Y_{1-x}Lu_x)AlO_4:Ln_z$$

$$(Ba_{1-x-y-2z}Sr_xCa_y)MgAl_{10}O_{17}:Ln_zNa_z$$

$$Ca_{1-2z}Al_4O_7:Ln_zNa_z$$

$$Sr_{4-2z}Al_{14}O_{25}:Ln_zNa_z$$

$$(Ba_{1-x-y-2z}Sr_xCa_y)Al_2O_4:Ln_zNa_z$$

$$Lu_{1-z}TaO_4:Ln_z$$

wherein

$$Ln = Pr, Nd, Sm, Er, Tm, Yb$$

$$x, y, a, b = 0.0 - 1.0$$

$$0.0 < z \leq 0.2$$

The luminescent compositions may therein be provided in the form of a monocrystalline structure, a polycrystalline structure, a polycrystalline ceramic structure, a micro-scale powder and/or a nano-scale powder.

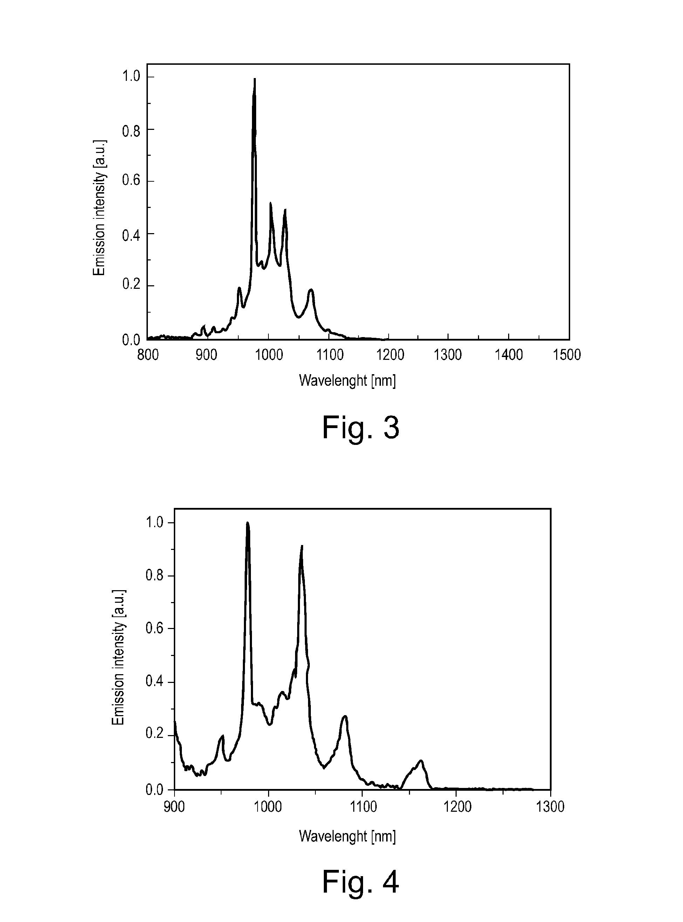

Fig. 3 shows an emission spectrum of a luminescent material which may be used for the wavelength converting element. In this case, the luminescent material is $Gd_2O_3$:Yb (Gadoliniumoxid-Ytterbium). This material has a maximum radiation emission at $\lambda_{max}$=967nm. The wavelength range around 967nm has to be removed from the emitted spectrum using e.g. a filter such that only the maxima at around 1007nm and around 1028nm remain.

Fig. 4 shows an emission spectrum for an alternative luminescent material in the form of $Lu_2O_3$:Yb (Lutetiumoxid-Ytterbium) having a maximum emission at $\lambda_{max}$=967 and 1034 nm. Again, the wavelength range around 967nm has to be removed from the emitted spectrum using e.g. a filter behind the screen such that only the maximum at around 1034nm remains.

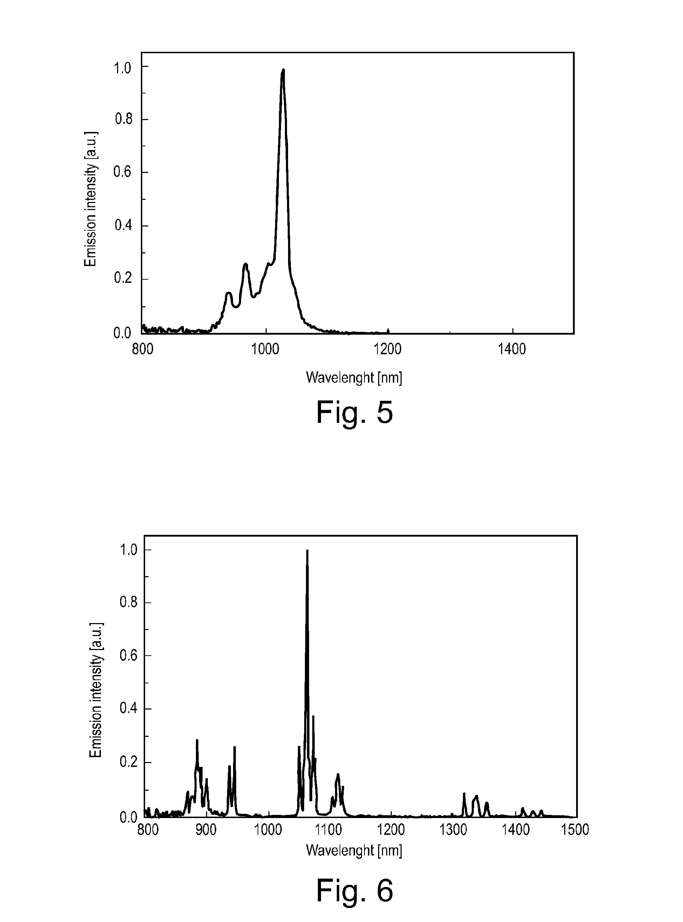

Fig. 5 shows the emission spectrum of an alternative luminescent material in the form of $Y_3Al_5O_{12}$:Yb (Yttrium-Aluminium-Granat-Ytterbium) having a maximum emission at $\lambda_{max}$=1025 nm.

Fig. 6 shows the emission spectrum of a further alternative luminescent material in the form of $Y_3Al_5O_{12}$:Nd (Yttrium-Aluminium-Granat-Neodymium) having a maximum emission at $\lambda_{max}$=1064 nm.

The light emitter 3 and the wavelength converting element 5 can be adapted such that the light source 1 emits infrared light with a power density of e.g. between 1 and 10000 mW/cm². With such power densities, the light source can be used to efficiently irradiate e.g. the skin of a person to thereby homogeneously heat the skin as well as possibly adjacent deeper tissue.

Thereby, beneficial medical effects such as increased oxygen partial pressure and improved blood perfusion may be obtained helping in e.g. curing of traumata, reduction of pain or treatment of warts. Due to the specific wavelength characteristics of the irradiated NIR radiation, patient's comfort and health may be reliably preserved.

It should be noted that the term "comprising" and similar does not exclude other elements or steps and that the indefinite article "a" does not exclude a plurality of items. Also elements described in association with different embodiments may be combined. It should be furthermore noted that reference signs in the claims shall not be construed as limiting the scope of the claims.

List of reference signs:

1    Light source

3    Light emitter

5    Wavelength conversion element

7    Housing

9    LED

11    Substrate

13    Supply line

15    Screen

17    non-infrared radiation

19    infrared radiation

**Claims**

1.  Light source (1) for emitting infrared radiation comprising:

    a light emitter (3) emitting non-infrared radiation (17);
    a wavelength converting element (5), which converts non-infrared radiation (17) emitted by the light emitter (3) into infrared radiation (19).

2.  Light source of claim 1, wherein the wavelength converting element (5) converts non-infrared radiation into infrared radiation within a wavelength spectrum excluding wavelength ranges in which water has maximum absorption of infrared radiation.

3.  Light source of one of claims 1 or 2, wherein the wavelength converting element (5) converts non-infrared radiation into infrared radiation within a wavelength spectrum consisting of at least one of a first range from below 950nm, a second range from 1000 to 1100nm, a third range from 1200 to 1350nm and a fourth range from 1500 to 1700nm.

4.  Light source of one of claims 1 to 3, wherein the wavelength converting element (5) comprises a luminescent material.

5.  Light source of claim 4, wherein the luminescent material comprises activator ions integrated into a matrix material.

6.  Light source of claim 5, wherein the activator ions comprise at least one of $Fe^{3+}$, $Pr^{3+}$, $Nd^{3+}$, $Sm^{3+}$, $Er^{3+}$, $Tm^{3+}$ and $Yb^{3+}$

7.  Light source of one of claims 4 to 6, wherein the luminescent material is provided in the form of at least one of a monocrystalline structure, a polycrystalline structure, a polycrystalline ceramic structure, a micro-scale powder and a nano-scale powder.

8.  Light source of one of claims 4 to 7, wherein the luminescent material is provided with at least one of

    $$AE_{1-2z}(Ti_{1-a-b}Zr_aHf_b)O_3:Ln_zNa_z$$

    $$(La_{1-x-y-z}Gd_xY_y)_2(Ti_{1-a-b}Zr_aHf_b)_2O_7:Ln_z$$

    $$(La_{1-x-y-z}Gd_xY_y)_2O_3:Ln_z$$

    $$(Lu_{1-x-y-z}Gd_xY_y)_3A1_5O_{12}:Ln_z$$

    $$(La_{1-x-y-z}Gd_xY_y)MgAl_{11}O_{19}:Ln_z$$

    $$(La_{1-x-y-z}Gd_xY_y)AlO_3:Ln_z$$

    $$Sr_{1-2z}Al_{12}O19:Ln_zNa_z$$

    $$Ca(Y_{1-x}Lu_x)AlO_{4:}Ln_z$$

    $$(Ba_{1-x-y-2z}Sr_xCa_y)MgAl_{10}O_{17:}Ln_zNa_z$$

    $$Ca_{1-2z}Al_4O_7:Ln_zNa_z$$

    $$Sr_{4-2z}Al4O_{25}:Ln_zNa_z$$

    $$(Ba_{1-x-y-2z}Sr_xCa_y)A1_2O_4:Ln_zNa_z$$

$$Lu_{1-z}TaO_{4:}Ln_Z$$

wherein

$$Ln = Pr, Nd, Sm, Er, Tm, Yb \text{ and } x, y, a, b = 0.0 - 1.0 \text{ and } 0.0 < z \leq 0.2.$$

9.  Light source of one of claims 5 to 8, wherein the matrix material comprises at least one of a crystalline material, a ceramic material, a glass-ceramic material, a glass-like material and a polymer material.

10. Light source of one of claims 5 to 9, wherein the activator ions are incorporated into a crystalline environment with at least one of sesquioxides, aluminates, garnets, sulfides, selenides, tellurides, titanates, zirconates, hafnates, tungstates, molybdates, and fluorides.

11. Light source of one of claims 1 to 10, wherein the wavelength converting element (5) is provided as a screen (15) being one of transparent and translucent and being arranged in a light path from the light emitter (3).

12. Light source of one of claims 1 to 12, wherein the light emitter (3) comprises at least one LED (9).

13. Light source of claim 12, wherein the light emitter (3) comprises an LED panel with a plurality of LEDs (9).

14. Light source of one of claims 1 to 13, wherein the light emitter (3) and the radiation converting element (5) are adapted such that the light source (1) emits infrared radiation with a power density of between 1 and 10000 mW/cm$^2$.

15. Use of a light source (1) of one of claims 1 to 14 for irradiating living tissue.

Fig. 1

Fig. 2

Fig. 3

Fig. 4

Fig. 5

Fig. 6

Europäisches
Patentamt

European
Patent Office

Office européen
des brevets

# EUROPEAN SEARCH REPORT

Application Number

EP 10 16 0399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 705 476 A1 (FUJI PHOTO FILM CO LTD [JP]; FUJINON CORP [JP]) 27 September 2006 (2006-09-27) * page 4 - page 11; claims; figures; examples * | 1-15 | INV.<br>C09K11/77<br>A61N5/00<br>A61N5/06<br>H01L33/00 |
| X | US 2008/149958 A1 (REEH ULRIKE [DE] ET AL REEH ULRIKE [DE] ET AL) 26 June 2008 (2008-06-26) * page 1 - page 4 * * page 6 - page 7; figures; examples * | 1,2,4,5, 7,9-14 | |
| X | J.STONE ET.AL.: "Nd:YAG single crystal fiber laser: room-temperature cw operation using a single LED as an end pump" APPLIED PHYSICS LETTERS, vol. 29, no. 1, 1976, pages 37-39, XP002596500 * page 37 - page 39 * | 1-4,6-8, 10,12,14 | |
| X | S.G. BOWN: "Phototherapy of tumors" WORLD J. SURG., vol. 7, 1983, pages 700-709, XP002596501 * page 701 * * page 703 - page 707 * | 1-4,6-8, 10,14,15 | TECHNICAL FIELDS SEARCHED (IPC)<br><br>C09K<br>A61N<br>H01L |
| X | LUO XIXIAN, CAO WANGHE: "Blue, green red upconversion luminescence and optical charactristics of rare earth doped rare earth oxide and oxysulfide" SCI CHINA SER B-CHEM, vol. 50, no. 4, 2007, pages 505-513, XP002596502 * page 506 - page 512 * | 1-15 | |

-/--

The present search report has been drawn up for all claims

| Place of search | Date of completion of the search | Examiner |
|---|---|---|
| Munich | 13 August 2010 | Doslik, Natasa |

EPO FORM 1503 03.82 (P04C01)

Europäisches
Patentamt

European
Patent Office

Office européen
des brevets

# EUROPEAN SEARCH REPORT

**Application Number**

EP 10 16 0399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI YANHONG ET.AL.: "Preparation and upconversion luminescence of nanocrystalline Gd2O3:Er,Yb" JOURNAL OF WUHAN UNIVERSITY OF TECHNOLOGY-MATER. SCI.ED., vol. 23, no. 4, 2008, pages 448-451, XP002596503 * page 448 - page 450 * | 1-3,6-8, 10 | |

----- 

**TECHNICAL FIELDS SEARCHED (IPC)**

The present search report has been drawn up for all claims

| Place of search | Date of completion of the search | Examiner |
|---|---|---|
| Munich | 13 August 2010 | Doslik, Natasa |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

................................................................

& : member of the same patent family, corresponding
　　document

EPO FORM 1503 03.82 (P04C01)

**ANNEX TO THE EUROPEAN SEARCH REPORT
ON EUROPEAN PATENT APPLICATION NO.**                    EP 10 16 0399

This annex lists the patent family members relating to the patent documents cited in the above-mentioned European search report.
The members are as contained in the European Patent Office EDP file on
The European Patent Office is in no way liable for these particulars which are merely given for the purpose of information.

13-08-2010

| Patent document cited in search report | | Publication date | Patent family member(s) | | Publication date |
|---|---|---|---|---|---|
| EP 1705476 | A1 | 27-09-2006 | US | 2006215170 A1 | 28-09-2006 |
| US 2008149958 | A1 | 26-06-2008 | US | 2010044739 A1 | 25-02-2010 |

For more details about this annex : see Official Journal of the European Patent Office, No. 12/82